# EUROPEAN PATENT APPLICATION

(11) **EP 3 714 702 A1**
(43) Date of publication of application: **30.09.2020**
(21) Application number: 19794034.9
(22) Date of filing: 04.04.2019
(51) Int. Cl.: A23L 33/135, A23K 10/10, A23K 20/163, A23L 2/38, A23L 2/52, A23L 33/125, A61K 8/73, A61K 8/99, A61K 31/715, A61K 35/747, A61P 37/08, A61Q 1/00

(54) **COMPOSITION FOR TYPE I ALLERGY**

(30) Priority: 25.04.2018 JP 2018084047
(71) Applicant: Sone Farm Co., Ltd., Tokyo 160-0022 (JP)
(72) Inventor: SUGIYAMA,Masanori, Hiroshima-shi, Hiroshima 732-0063 (JP)
(74) Representative: Papula Oy
(86) International application number: PCT/JP2019/014996
(87) International publication number: WO 2019/208149

(57) **Abstract**

A composition comprises, as an active ingredient, a cell body of a lactic acid bacterium belonging to *Lactobacillus paracasei,* a cultured product or fermented product thereof, or a polysaccharide produced thereby is effective in preventing or improving type I allergy and can be used as a food or drink, a medicine, a feed, and a cosmetic product, for the prevention or improvement of particularly anaphylaxis.

## Description

### Technical Field

The present invention relates to a composition for type I allergy. More specifically, the present invention relates to a composition for type I allergy, particularly for the prevention or improvement of anaphylaxis, comprising, as an active ingredient, a cell body of a lactic acid bacterium belonging to *Lactobacillus paracasei,* a cultured product or fermented product thereof, or a polysaccharide produced thereby.

### Background Art

In recent years, it has been pointed out that allergies including hay fever, atopic dermatitis, and contact dermatitis are remarkably increased due to various causes such as changes of diet, deterioration of air pollution, allergens, and exposure to metallic decorations. Allergies are regarded as a systemic or local failure of a living body based on an immune response. Allergies are broadly classified into type I, II, and III allergies based on a humoral immune reaction caused by blood antibodies, and type IV allergy based on a cell-mediated immune reaction caused by sensitized lymphocytes. Type I allergy is considered to be caused by conditions that antigen-presenting cells are biased in the differentiation of T cells when an antigen such as pollen enters the body, whereby B cells induce the production of IgE antibodies. Anaphylaxis is a systemic disease classified as type I allergy and sometimes falls into a life-threatening shock state (anaphylactic shock).

Drugs used for such allergies include antihistamines and steroids. It has, however, been pointed out that these drugs show various side effects, and they cannot be deemed to be always safe.

Under these circumstances, in recent years, lactic acid bacteria that are involved in an immune reaction and have an antiallergic action have attracted attention as a highly safe antiallergic material. For example, proposals have been made on lactic acid bacteria, such as *Lactobacillus brevis,* that suppress the production of IgE antibodies (Patent Document 1), a lactic acid bacterium of *Lactobacillus reuteri* (strain AD0002) having an effect of suppressing histamine release (Patent Document 2), a lactic acid bacterium of *Enterococcus faecium* that activates an intestinal immunity or type I helper T cells (Patent Documents 3 and 4), a lactic acid bacterium of *Lactobacillus paracasei* strain KW3110 having a Th1 immunity enhancing action and a Th2 immunosuppressive action (Non-Patent Document 1), and the like.

### Prior Art Documents

### Patent Documents

Patent Document 1: JPH 09-2959 A
Patent Document 2: JP 2000-95697 A
Patent Document 3: JP 2006-67881 A
Patent Document 4: JP 2006-104107 A

### Non-Patent Documents

Non-Patent Document 1: International archives of allergy and immunology 2004; 135;205-215

### Summary of Invention

### Problem to be solved by invention

Under such background arts, it has been desired to develop a new composition for anti-allergies containing a lactic acid bacterium as an active ingredient. The problem to be solved by the present invention is thus to provide a new composition for type I allergy, particularly effective in the prevention or improvement of anaphylaxis, comprising a lactic acid bacterium as an active ingredient.

### Means for solving the problem

The present inventor has made conducted intensive studies with the aim of developing a new composition for type I allergy, particularly for the prevention or improvement of anaphylaxis. As a result, the present inventor has found that a lactic acid bacterium belonging to *Lactobacillus paracasei* has an action of suppressing mouse active cutaneous anaphylaxis and an action of preventing or improving type I allergy, particularly anaphylaxis. On the basis of these findings, the present inventor has further studied and completed the present invention.

In one aspect of the present invention, the present invention relates to a composition for the prevention or improvement of type I allergy, comprising, as an active ingredient, a cell body of a lactic acid bacterium belonging to *Lactobacillus paracasei,* a cultured product or fermented product thereof, or a polysaccharide produced thereby.

The composition of the present invention can be preferably used to prevent or improve anaphylaxis.

In the composition of the present invention, the lactic acid bacterium is preferably a lactic acid bacterium derived from a fig, and particularly preferably *Lactobacillus paracasei* strain IJH-SONE68 (Accession No. NITE BP-02242) or a lactic acid bacterium equivalent thereto.

In the composition of the present invention, a cultured product or fermented product of the lactic acid bacterium is preferably the one that can be obtained by cultivating or fermenting the lactic acid bacteria in the presence of a fruit juice of pineapple genus plant.

In the composition of the present invention, a polysaccharide produced by the lactic acid bacterium includes a neutral polysaccharide having a structure in which N-acetylglucosamines are linked with each other via α-1,6 bond, and an acidic polysaccharide composed mainly of glucoses and mannoses.

The composition of the present invention is preferably a food or drink composition, and the food or drink includes a beverage, a functional food, a fermented food, and a supplement.

In addition, the composition of the present invention is preferably a pharmaceutical composition.

Furthermore, the composition of the present invention is preferably a feed composition or a cosmetic composition.

### Effect of Invention

The composition of the present invention has an action of suppressing mouse active cutaneous anaphylaxis, is effective in the prevention or improvement of type I allergy and can be used to prevent or improve particularly anaphylaxis. The composition of the present invention can be used as a food or drink, a medicine, a feed, and a cosmetic product, for the prevention or improvement of type I allergy such as anaphylaxis. The composition of the present invention comprises, as an active ingredient, a cell body of a lactic acid bacterium belonging to *Lactobacillus paracasei,* a cultured product or fermented product thereof, or a polysaccharide produced thereby. Therefore, the composition of the present invention has high safety, can be applied for a long period of time, can be supplied in a large amount at low cost, and has extremely high utility and practicality.

### Brief Description of Drawings

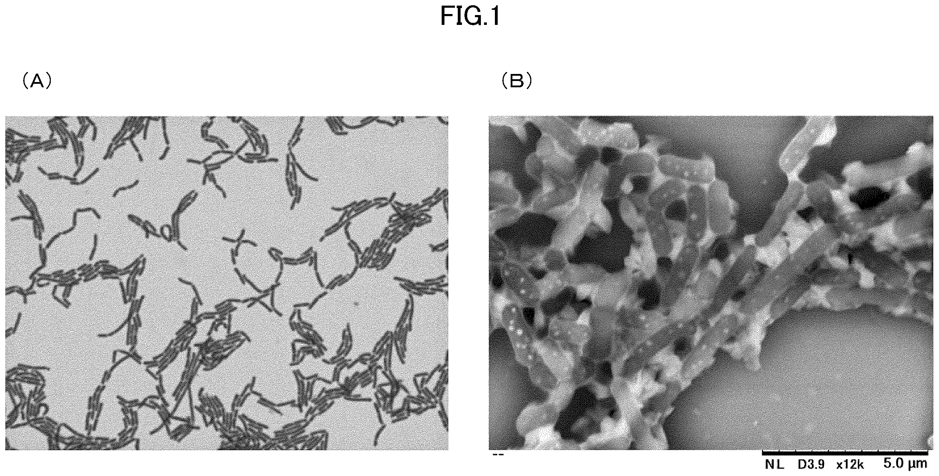

[Fig. 1] Fig. 1 shows microscope photographs of *Lactobacillus paracasei* strain IJH-SONE68. (A) in Fig. 1 is a gram-stained microscope photograph, and (B) in Fig. 1 is a scanning electron microscope (SEM) photograph.

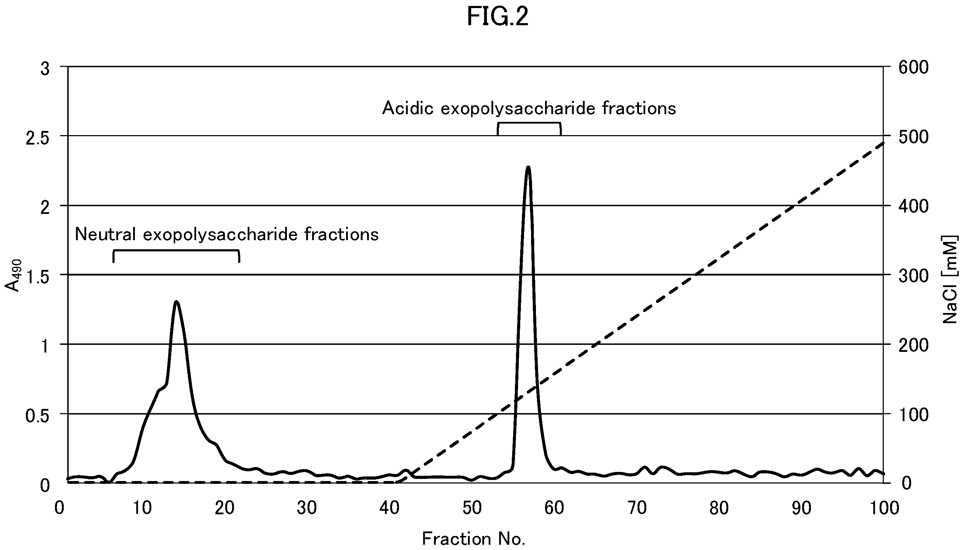

[Fig. 2] Fig. 2 illustrates an isolation profile of anion exchange chromatography (TOYOPEARL DEAE-650M resin (Tosoh Corporation)) of exopolysaccharides from *Lactobacillus paracasei* strain IJH-SONE68. The exopolysaccharides were eluted with NaCl having a gradient concentration of 0 mM to 500 mM (broken line), and the exopolysaccharides in each fraction were monitored at 490 nm by a phenol sulfuric acid method (straight line).

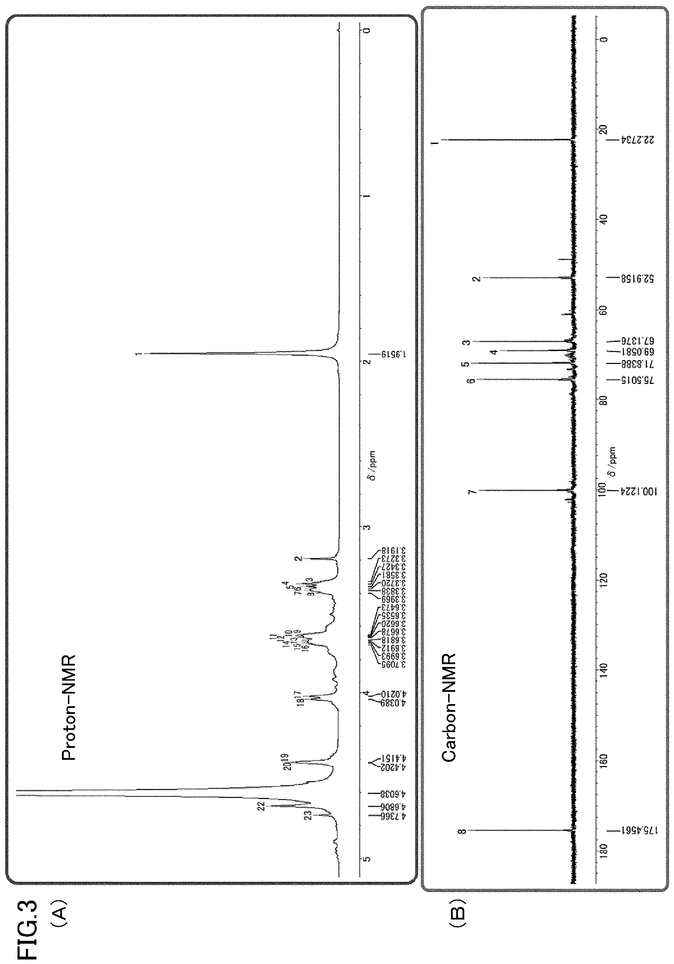

[Fig. 3] Fig. 3 illustrates each NMR profile obtained by subjecting a neutral exopolysaccharide, which was obtained by purifying exopolysaccharides from *Lactobacillus paracasei* strain IJH-SONE68 with anion exchange column chromatography, to proton-NMR and carbon-NMR. (A) in Fig. 3 is the NMR profile of proton-NMR, and (B) in Fig. 3 is the NMR profile of carbon-NMR.

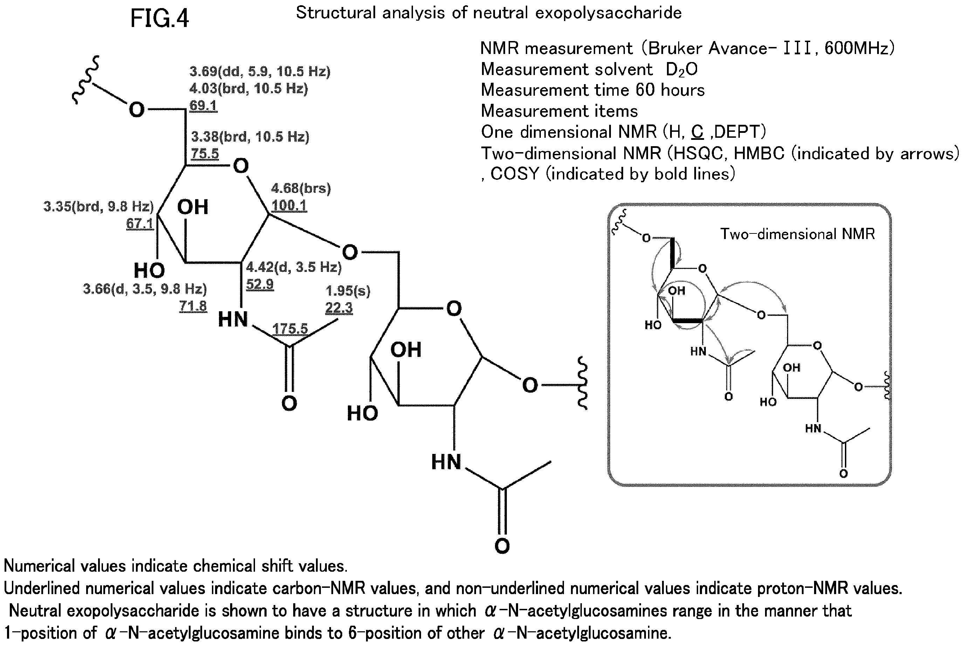

[Fig. 4] Fig. 4 illustrates results of structurally analyzing a neutral exopolysaccharide on the basis of the NMR profiles. These structural analysis results revealed that the neutral exopolysaccharide of *Lactobacillus paracasei* strain IJH-SONE68 has a structure in which N-acetylglucosamines are linked with each other via α-1,6 bond.

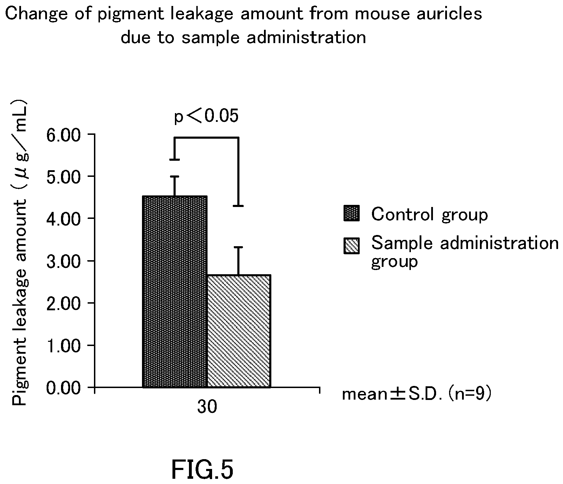

[Fig. 5] Fig. 5 is a graph illustrating that a fermented solution of *Lactobacillus paracasei* strain IJH-SONE68 has an action of suppressing cutaneous anaphylaxis that has actively been induced in mice.

### Embodiments for Carrying out Invention

The following detailed disclosures are made on the composition provided by the present invention for the prevention of improvement of type I allergy, comprising, as an active ingredient, a cell body of a lactic acid bacterium belonging to *Lactobacillus paracasei,* a cultured product or fermented product thereof, or a polysaccharide produced thereby.

### 1. The lactic acid bacterium in the present invention

The lactic acid bacterium in the present invention is a lactic acid bacterium belonging to *Lactobacillus paracasei* and preferably a lactic acid bacterium derived from a fig. Specifically, the lactic acid bacterium includes *Lactobacillus paracasei* strain IJH-SONE68 that was isolated and identified from leaves of a fig according to the present invention. This strain was nationally deposited under the accession number of NITE P-02242 at Patent Microorganisms Depositary, National Institute of Technology and Evaluation (#122, 2-5-8 Kazusakamatari, Kisarazu-shi, Chiba 292-0818, Japan) on April 19, 2016. The deposition was then transferred to an international deposit under the Budapest Treaty and given the international deposit accession number of NITE BP-02242 on May 26, 2017.

As illustrated in the photograph of Fig. 1, *Lactobacillus paracasei* strain IJH-SONE68 is a catalase-negative, gram-positive bacillus, and has mycological properties of forming a white colony and the mycological characteristic of conditional heterolactic fermentation. Furthermore, the strain has an ability to produce polysaccharides.

A lactic acid bacteria equivalent to *Lactobacillus paracasei* strain IJH-SONE68 is also included in the present invention. Here, the equivalent lactic acid bacterium indicates a lactic acid bacterium that belongs to *Lactobacillus paracasei* and has an action of suppressing type I allergy, particularly anaphylaxis, like *Lactobacillus paracasei* strain IJH-SONE68. The equivalent lactic acid bacterium is obtained, for example, by performing usual mutation treatment technique, such as mutation and genetic recombination, on *Lactobacillus paracasei* strain IJH-SONE68 and, in addition, may be a bacterial strain that has been bred by selecting natural mutation strains of *Lactobacillus paracasei* strain IJH-SONE68, and the like.

### 2. The active ingredient in the present invention

The composition of the present invention contains, as an active ingredient, a cell body of the above-mentioned lactic acid bacterium, a cultured product or fermented product thereof, or a polysaccharide produced thereby.

The lactic acid bacterium can be cultured by a commonly used culture method such as liquid stationary culture, using a commonly used MRS medium or a modified medium thereof. The lactic acid bacterium can promote its growth by the culture in the presence of a fruit juice of a pineapple genus plant or its extract (WO 2011/046194 A). In addition, the lactic acid bacterium can also promote its growth by the culture in the presence of sake lees, sake lees extract or sake lees enzymes (JPH 03-172171 A, JPH 05-15366 A, and JP 2835548 B). As the lactic acid bacterium, a culture obtained after the cultivation may be used as it is, the obtained culture solution may be diluted or concentrated to be used, or the cell body recovered from the culture may be used. In addition, as long as the effect of the present invention is not impaired, various additional operations such as heat and freeze-dry may also be performed after the cultivation. The cell body of the lactic acid bacterium may be a viable or dead cell body, in which polysaccharides are adhered to the cell surface of the lactic acid bacterium, and may include both the viable and dead cell bodies. The dead cell body may be crushed and preferably has polysaccharides adhered to the cell surface thereof. In addition, the fermented product of the lactic acid bacterium can be obtained by fermenting the lactic acid bacterium usually using glucose or the like as a nutrient source, and further using yeast extract, fruit juice of pineapple genus plants, sake lees, distilled spirit lees, etc., if necessary.

Polysaccharides produced by the lactic acid bacterium can be obtained by isolating and purifying them from a culture of lactic acid bacteria belonging to *Lactobacillus paracasei* according to an ordinary method. Specifically, for example, the polysaccharides can be obtained by removing the cell body from a culture of lactic acid bacteria belonging to *Lactobacillus paracasei* by centrifugation, and precipitating polysaccharides from the obtained culture using ethanol, acetone, or the like. In addition, the polysaccharides can be obtained by further isolating and purifying them by ion exchange chromatography.

In the present invention, the polysaccharides produced by the lactic acid bacterium specifically include a neutral polysaccharide having a structure in which N-acetylglucosamines are linked with each other via α-1,6 bond, and an acidic polysaccharide composed mainly of glucoses and mannoses. This neutral polysaccharide can be obtained by isolating and purifying polysaccharides obtained from a culture of *Lactobacillus paracasei* strain IJH-SONE68 by anion exchange chromatography, as disclosed in Example 2 herein below. It was revealed from the proton-NMR and carbon-NMR profiles illustrated in Fig. 3 that this neutral polysaccharide has a structure in which N-acetylglucosamines are linked with each other by α-1,6 bonds. In addition, the *Lactobacillus paracasei* strain IJH-SONE68 secretes an acidic polysaccharide mainly composed of glucose and mannose outside the cell body. More specifically, this acidic polysaccharide is composed of glucose, mannose, galactose, and rhamnose, and their composition ratio is approximately 10:170: 2:1.

### 3. The composition in the present invention

The composition of the present invention can be used in various forms of a food or drink composition, a pharmaceutical composition, a feed composition, and a cosmetic composition.

The food or drink of the food or drink composition are not particularly limited but include beverages such as soft drinks, carbonated drinks, nutritional drinks, fruit juice beverages, and lactic acid bacteria beverages, concentrated stock solutions of these beverages, powders for the preparation of these beverages, and the like; ice cream, sherbet and ice confectionery such as shaved ice; confectioneries such as candy, gummy, cereal, chewing gum, candy, gum, chocolate, tablet candy, snack, biscuit, jelly, jam, cream, and baked confectionery; dairy products such as processed milk, milk drink, fermented milk, drink yogurt, and butter; bread; enteral nutritious food, liquid food, childcare milk, sports drink; food such as puree; seat sake; and other functional foods. In addition, the food or drink may be supplements, and the supplements may be in the form of, for example, granules, powders, or tablets.

The food or drink as disclosed above may be prepared by adding a cell body of a lactic acid bacterium belonging to *Lactobacillus paracasei,* a cultured product or fermented product thereof, or a polysaccharide produced thereby, to raw materials of food or drink, or prepared in the same manner as an usual food or drink. The addition of a cell body of a lactic acid bacterium belonging to *Lactobacillus paracasei,* a cultured product or fermented product thereof, or a polysaccharide produced thereby may be performed at any stage of the process of preparing the food or drink. The food or drink may be prepared after a fermentation process of the added lactic acid bacteria. Examples of such food or drink include fermented foods such as lactic acid bacterium beverages and fermented milks.

The content of a cell body of the lactic acid bacterium or a cultured product or fermented product thereof in the food or drink composition may be appropriately determined depending on the embodiment of the food or drink, but is the one so that a cell body of the lactic acid bacterium is usually contained in the food or drink composition preferably in the range of 1×10⁶ to 1×10¹² cfu/g or 1×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the food or drink composition at an amount of 0.001% or more by weight, preferably at an amount of 0.01% or more by weight, in terms of the weight of the polysaccharide.

The pharmaceutical composition is usually used by blending a cell body of the lactic acid bacterium, a cultured product or fermented product thereof or a polysaccharide produced thereby with a physiologically acceptable liquid or solid of a pharmaceutical carrier usually used, followed by the formulation. The dosage form of the pharmaceutical composition is not particularly limited, but includes tablets, pills, powders, solutions, suspensions, emulsions, granules, capsules, syrups, suppositories, injections, ointments, patches, eye drops, and nose drops.

The content of a cell body of the lactic acid bacterium or a cultured product or fermented product thereof in the pharmaceutical composition may be appropriately determined depending on the dosage form, the dosage regimen, the age and sex of a subject, the kind of disease, the degree of disease, other conditions and the like, but is the one so that a cell body of the lactic acid bacterium is usually contained in the pharmaceutical composition preferably in the range of 1×10⁶ to 1×10¹² cfu/g or 1×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the pharmaceutical composition at an amount of 0.001% or more by weight, preferably at an amount of 0.01% or more by weight, in terms of the weight of the polysaccharide.

The administration timing of the pharmaceutical composition of the present invention is not particularly limited but may be appropriately chosen depending on a subject to be applied. The pharmaceutical composition may also be administered prophylactically or used in a maintenance therapy. The administration mode may be preferably appropriately determined depending on the dosage form, age, sex and other conditions of the administered subject, the degree of symptoms of the administered subject, and the like. In any case, the pharmaceutical composition of the present invention may be administered once per day, administered dividedly into two or more times or administered once every several days or weeks.

Examples of the feed of a feed composition include pet food, livestock feed and fish feed. Such a feed may be prepared by mixing common feed, for example, cereals, cakes, brans, fish meals, bone meals, oils and fats, skim milk powders, wheys, bitterns, mineral feeds, yeasts, or the like with a cell body of the lactic acid bacterium, a cultured product or fermented product thereof or a polysaccharide produced thereby. In addition, for example, like the case of silage, a feed may be prepared through a fermentation process with the lactic acid bacterium added thereto. The prepared feed may be orally administered to general mammals, livestock, farmed fishes, pet animals or the like. In the case of farmed fishes, it may be adopted to spread fermented products, to which a cell body of the lactic acid bacterium, a cultured product or fermented product thereof or a polysaccharide produced thereby is added, to the farmed place of fishes.

The content of a cell body of the lactic acid bacterium or a cultured product or fermented product thereof in the feed composition may be appropriately determined depending on the embodiment of the feed or the administered subject, but is the one so that a cell body of the lactic acid bacterium is usually contained in the feed composition preferably in the range of 1×10⁶ to 1×10¹² cfu/g or 1×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the polysaccharide produced by the lactic acid bacterium, the polysaccharide is usually contained in the feed composition at an amount of 0.001% or more by weight, preferably at an amount of 0.01% or more by weight, in terms of the weight of the polysaccharide.

Examples of the cosmetic product of the cosmetic composition containing the lactic acid bacterium of the present invention include washing agents such as soaps, body shampoos, cleansing creams, and facial cleansers; creams such as lotions, vanishing creams, cold creams, emollient creams, and massage creams; milky lotions and serums. The cosmetic composition of the present invention may be obtained by adding a cell body of the lactic acid bacterium of the present invention, a cultured product or fermented product thereof, or a polysaccharide produced thereby, to materials usually used in the above-mentioned cosmetic products.

In the cosmetic composition of the present invention, it is preferable to use, for example, a lactic acid-fermented egg white obtained by adding the lactic acid bacterium of the present invention to a liquid egg white prepared by breaking eggs of birds such as chickens and removing the egg yolk, followed by the fermentation. In general, such lactic acid fermentation is preferably performed by using glucose or the like as a nutrient source, adding a fermentation promoting substance such as yeast extract, if necessary, and fermenting them. The form of the lactic acid-fermented egg white may be, for example, liquid, powder, cream, paste or jelly, depending on the cosmetic product to be blended therewith.

The content of a cell body of the lactic acid bacterium or a cultured product or fermented product thereof in the cosmetic composition of the present invention may be appropriately determined depending on the embodiment of the cosmetic product or the applied subject, but is the one so that a cell body of the lactic acid bacterium is usually contained in the cosmetic composition preferably in the range of 1×10⁶ to 1×10¹² cfu/g or 1×10⁶ to 1×10¹² cfu/ml, more preferably in the range of 1×10⁷ to 1×10¹¹ cfu/g or 1×10⁷ to 1×10¹¹ cfu/ml. In the case where the lactic acid bacterium is a dead cell body, the cfu/g or cfu/ml can be replaced with the number of cells per g or the number of cells per ml. In the case of the lactic acid-fermented egg white, the content of the egg white is usually 0.001% or more by weight, preferably 0.01% or more by weight, in terms of the dried egg white. In the case of the polysaccharide produced by the lactic acid bacterium, the polysaccharide is usually contained in the cosmetic composition at an amount of 0.001% or more by weight, preferably at an amount of 0.01% or more by weight, in terms of the weight of the polysaccharide.

### 4. Use of the composition of the present invention

A cell body of a lactic acid bacterium belonging to Lactobacillus paracasei, a cultured product or fermented product thereof, or a polysaccharide produced thereby has an action of suppressing mouse active cutaneous anaphylaxis and an action of suppressing type I allergy, particularly anaphylaxis. Therefore, a cell body of a lactic acid bacterium belonging to *Lactobacillus paracasei,* a cultured product or fermented product thereof, or a polysaccharide produced thereby can be used to prevent or improve type I allergy including pollen allergy, allergic rhinitis, bronchial asthma, atopic dermatitis, itchy skin, and conjunctivitis. In particular, a cell body of a lactic acid bacterium belonging to *Lactobacillus paracasei,* a cultured product or fermented product thereof, or a polysaccharide produced thereby has an action of suppressing anaphylaxis and can be therefore used to prevent or improve anaphylaxis due to oral ingestion of food or the like, anaphylaxis due to non-invasive exposure of drugs or the like to skin or mucous membranes, anaphylaxis due to invasive exposure such as insect bites, and the like. In addition, the composition of the present invention is effective in the prevention or improvement of type I allergy and can be therefore used as materials for food or drink for the maintenance and enhancement of health.

### Examples

Hereinafter, the present invention will be disclosed in more detail with reference to examples, but the present invention is not limited by these examples.

### Example 1

### Isolation and identification of lactic acid bacterium

### 1. Isolation of lactic acid bacterium sample

The leaves, stems, and fruits of a fig (variety "TOYOMITSU HIME") were chosen and cut into pieces of 2 to 3 mm using sterilized tweezers and scissors. Every five to six pieces were each then placed in a sterilized test tube containing MRS liquid medium, and statically cultured at 28°C and 37°C until the MRS medium as a standard medium for a lactic acid bacterium became turbid (proliferated). By the way, it took 2 to 4 days for the proliferation of the lactic acid bacterium candidate strains to be visible.

A part of each culture liquid of the lactic acid bacterium candidate strains was subjected to a line drawing paint on MRS agar medium using a disposable loop, followed by stationary culture. Among colonies formed on the agar medium, all of differently colored, lustrous and shaped colonies were picked up and subjected to a line drawing paint on a fresh MRS agar medium, and the colonies were purified.

H₂O₂ test was performed for each purified colony to verify the presence or absence of the production of a catalase enzyme. This is a test method for observing the presence or absence of oxygen generated when catalase is present, which is observed when microbial bodies are exposed to 10% H₂O₂ solution. By the way, a lactic acid bacterium produces no catalase.

As a result of attempting the search and isolation from a fig, one lactic acid bacterium candidate strain showing catalase-negative was obtained from the leaves of a fig as the isolation source.

### 2. Identification of the isolated strain

The aforementioned lactic acid bacterium candidate strain was again cultured in MRS liquid medium, and the microbial bodies were obtained by centrifugation. After the microbial bodies were treated with cell wall lytic enzyme, a genomic DNA was extracted using DNAzol reagent.

According to the method as disclosed in Lane, DJ (1991), "16S/23S rRNA sequencing", Nucleic Acid Techniques in Bacterial Systematics, pp. 115-175, edited by E. Stackebrandt & M. Goodfellow. Chichester: Wiley, a genomic DNA PCR was performed using a genomic DNA as a template and using 27f primer and 1525r primer, thereby to amplify 16S rDNA part. Then, an objective fragment was recovered from agarose gel according to NucleoSpin Gel and PCR Clean-up kit (manufactured by Mahalay Nagel). A sequencing reaction by a dye terminator method for sequencing a base sequence was performed with Big Dye Terminator Cycle Sequencing FS Ready Reaction Kit ver. 3.1 (manufactured by ThermoFisher Scientific), and analysis was made with ABI PRISM 3130x1 Genetic Analyzer (manufactured by ThermoFisher Scientific). The base sequence of the analyzed 16S rDNA was subjected to a homology search by BLAST program and compared with the database of DNA data bank (DDBJ/EMBL/GenBank) to make a taxonomic identification on the isolated strain.

The lactic acid bacterium candidate strain isolated from leaves of a fig was named strain IJH-SONE68 and identified as *Lactobacillus paracasei* because it was 100% identical to a base sequence which was in the strain of *Lactobacillus paracasei* R094 already registered in DNA data bank (DDBJ/EMBL/GenBank) and which had NR-025880 as the accession number of the base sequence.

This strain was nationally deposited under the accession number of NITE P-02242 at Patent Microorganisms Depositary, National Institute of Technology and Evaluation (#122, 2-5-8 Kazusakamatari, Kisarazu-shi, Chiba 292-0818, Japan) on April 19, 2016. The deposition was then transferred to an international deposit under the Budapest Treaty and given the international deposit accession number of NITE BP-02242 on May 26, 2017.

### 3. Mycological properties of separated and identified lactic acid bacterium

The aforementioned isolated and identified lactic acid bacterium strain IJH-SONE68 was a catalase-negative, gram-positive rod and had a white colony forming property, as shown in the photograph of Fig. 1, and further had the characteristic of conditional hetero-lactic acid fermentation and the ability of producing polysaccharides.

### 4. Saccharide assimilation ability of the isolated and identified lactic acid bacterium

### (1). Test method of assimilation ability

The strain IJH-SONE68 was investigated for the assimilation ability of 49 kinds of saccharides according to the following test method.

The strain IJH-SONE68 was statically cultured in MRS liquid medium until the proliferation stationary phase. The cell bodies obtained by centrifugation were washed with an appropriate amount of a suspension medium (manufactured by BioMeieux), and finally suspended in 2 mL of a suspension medium. A portion of the resultant suspension was added to 5 mL of a suspension medium to determine an amount (n) for McFarland turbidity to become 2. Subsequently, 2n of a microbial solution was added to API 50 CHL medium (manufactured by BioMerieux), and this solution was dispended to each well of API 50 CHL kit (manufactured by BioMerieux, 49 kinds of saccharides were coated on the bottom of each well). Finally, mineral oil was overlaid and set in a tray containing a sterilized water. After culturing at 37°C for 48 hours, the presence or absence of the assimilation ability was assessed by observing the change in color tone in each well.

### 5. Test results of the assimilation ability

Table 1 shows the results of investigating the assimilation ability of the strain IJH-SONE68 against 49 kinds of saccharides.

**[Table 1]**

| Assimilation abilities of the strain IJH-SONE68 against saccharides | |
|---|---|
| Substrates | Assimilation abilities |
| Control | - |
| Glycerol | - |
| Erythritol | - |
| D-Arabinose | - |
| L-Arabinose | - |
| D-Ribose | + |
| D-Xylose | - |
| L-Xylose | - |
| D-Adonitol | + |
| Methy - βD - xylopyranoside | - |
| D-Galactose | + |
| D-Glucose | + |
| D-Fructose | + |
| D-Mannose | + |
| L-Sorbose | + |
| L-Rhamnose | - |
| Dulcitol | - |
| Inositol | - |
| D-Mannitol | + |
| D-Sorbitol | - |
| Methyl-αD-mannopyranoside | - |
| Methyl-αD-glucopyranoside | - |
| N-Acetylglucosamine | + |
| Amygdalin | - |
| Arbutin | - |
| Esculin + Ferric citrate | + |
| Salicin | + |
| D-Cellobiose | + |
| D-Maltose | + |
| D-Lactose | - |
| D-Melibiose | - |
| D-Sucrose | + |
| D - Trehalose | + |
| Inulin | - |
| D-Melezitose | + |
| D-Raffinose | - |
| Starch | - |
| Glycogen | - |
| Xylitol | - |
| Gentibiose | + |
| D-Turranose | - |
| D-Lyxose | - |
| D-Tagatose | + |
| D-Fucose | - |
| L-Fucose | - |
| D-Arabitol | - |
| L-Arabitol | - |
| Gluconic acid | + |
| 2-Ketogluconic acid | - |
| 5-Ketogluconic acid | - |

| | |
|---|---|
| In Table 1, + indicates the possession of assimilation ability, and - indicates no possession of assimilation ability. | |

### Example 2

### 1. Isolation and purification of exopolysaccharides produced by the strain IJH-SONE68

Exopolysaccharides produced by the strain IJH-SONE68 were isolated and purified according to the following method.

The strain IJH-SONE68 was statically cultured in MRS liquid medium until the proliferation stationary phase. 5 mL of the resultant culture solution was used as a seed culture solution, and inoculated on 5 L of a semisynthetic medium for producing exopolysaccharides (the composition thereof will be disclosed below), followed by static culture at 37°C for 120 hours. After the resultant culture solution was cooled to 4°C, proteins contained in the culture supernatant were denatured, and 202.5 mL of a 100% trichloroacetic acid aqueous solution was added thereto, mixed and allowed to stand for 30 minutes to remove them as precipitates in a later step. After the precipitates were removed by centrifugation, an equal amount of acetone was added to the collected supernatant and mixed, and the resultant mixture was allowed to stand at 4°C overnight to precipitate polysaccharides produced by the strain IJH-SONE68. The precipitates were collected by centrifugation, and the resultant precipitates were then washed with 250 mL of 70% ethanol. After the precipitates were air-dried, 75 mL of 50 mM Tris-HCl buffer (pH 8.0) was added to the resultant precipitates and mixed for 1 hour to dissolve the precipitates. After insoluble impurities were removed by centrifugation to recover a supernatant, 750 µL of 1 mg/mL DNase solution (Worthington, Inc.) and 750 µL of 1 mg/mL RNase solution (Nacalai Tesque, Inc.) were each added to the recovered supernatant, followed by being allowed to react at 37°C for 8 hours. Subsequently, 750 µL of 2 mg/mL proteinase K solution (manufactured by Wako Pure Chemical Industries, Ltd.) was added, and the resultant mixture was reacted at 37°C for 16 hours. The resultant solution after the reaction was cooled to 4°C, the added enzymes were each denatured, and 8.75 mL of a 100% trichloroacetic acid aqueous solution was then added thereto, mixed and allowed to stand for 1 hour to remove the enzymes as precipitates in the next centrifugation. The resultant precipitates were removed by centrifugation to obtain a supernatant, 262.5 mL of 100% ethanol was added to the obtained supernatant, the resultant mixture was thoroughly mixed, and the polysaccharides produced by the strain IJH-SONE68 strain were then recovered as precipitates by centrifugation. After the precipitates were washed with 50 mL of 70% ethanol, the precipitates were air-dried, an appropriate amount (about 25 mL) of a purified water was added thereto, and the resultant mixture was allowed to stand overnight at 4°C to dissolve the polysaccharides. For the polysaccharides sample after the dissolution, small molecules such as monosaccharides in the recovered sample were removed using an ultrafiltration unit (Merck Ltd.) of 10,000 MWCO while replacing the solvent with a purified water, and a purified polysaccharide sample was thus obtained.

The purified polysaccharide sample was applied to an open column (2.5 × 22 cm) packed with TOYOPEARL DEAE-650M resin (Tosoh Corporation) previously equilibrated with 50 mM Tris-HCl buffer (pH 8.0), and column work was performed to isolate and purify the sample to neutral polysaccharide fractions and acidic polysaccharide fractions. The same buffer was used as an elution solution, and a flow rate was fixed at 1 mL/min. In addition, eluates were collected in different test tubes at every 6 mL. First, from the beginning to 240 minutes, elution was made with the same buffer (Test Tube Nos. 1 to 40). Next, from 240 minutes to 600 minutes, a concentration gradient of 0 to 500 mM NaCl was prepared using the same buffer, and elution was continued with the gradient (Test Tube Nos. 41 to 100). The column isolation spectrum is illustrated in Fig. 2. After the presence of polysaccharides was confirmed by a phenol sulfuric acid method (disclosed below) for all the samples eluted in the test tubes, the confirmed solutions in the test tubes were collected as neutral polysaccharide fractions and acidic polysaccharide fraction, respectively. For each fraction, an ultrafiltration unit of 10,000 MWCO was used to remove small molecules such as monosaccharides in the recovered sample while replacing the solvent with purified water.

As a result, neutral polysaccharide fractions and acidic polysaccharide fractions were isolated and purified as exopolysaccharides produced by the strain IJH-SONE68.

A semisynthetic medium for producing polysaccharides was prepared by modifying a medium disclosed in Kimmel SA, Roberts RF., "Development of a growth medium suitable for exopolysaccharide production by Lactobacillus delbrueckii ssp. Bulgaricus RR.", Int. J. Food Microbiol., 40, 87-92 (1998), as follows:

| Semisynthetic medium for producing polysaccharides | [g/L] |
|---|---|
| Glucose | 20 |
| Tween 80 | 1.0 |
| Ammonium citrate | 2.0 |
| Sodium acetate | 5.0 |
| MgSO₄•7H₂O | 0.1 |
| MnSO₄•5H₂O | 0.05 |
| K₂HPO₄ | 2.0 |
| Bacto casitone | 10.0 |
| Vitamin Soln. | 2 mL |
| Trace element Soln. | 1 mL |

| Vitamin Soln. | [g/L] |
|---|---|
| 4-Aminobenzoic acid | 0.05 |
| Biotin | 0.001 |
| Folic acid | 0.025 |
| Lipoic acid | 0.025 |
| Nicotinic acid | 0.1 |
| Pantothenic acid | 0.05 |
| Pyridoxamin-HCl | 0.25 |
| Vitamin B₁₂ | 0.05 |
| Pyridoxine | 0.025 |
| Riboflavin | 0.05 |
| Thiamine | 0.1 |

Trace element Soln. is disclosed in Kets EPW, Galinski EA, de Bont JAM. Carnitine: "A novel compatible solute in Lactobacillus plantarum", Arch. Microbiol., 192, 243-248 (1994), and the composition is as follows:

| Trace element Soln. | [g/L] |
|---|---|
| 25% HCl | 10 mL |
| FeCl₂ •4H₂O | 1.5 |
| CoCl₂ •6H₂O | 0.19 |
| MnCl₂ •4H₂O | 0.1 |
| ZnCl₂ | 0.07 |
| H₃BO₃ | 0.006 |
| Na₂MoO₄ •2H₂O | 0.036 |
| NiCl₂ •6H₂O | 0.024 |
| CuCl₂ •2H₂O | 0.002 |

Phenol sulfuric acid method (DuBois M, Gilles KA, Hamilton JK, Rebers PA, Smith F., "Colorimetric method for determination of sugars and related substances", Anal. Chem., 28, 350-356 (1956))

30 µL of a subject sample was mixed with an equal amount of 5 w/v% phenol aqueous solution, and 150 µL of a concentrated sulfuric acid was added to the resultant mixture and mixed with each other to allow a reaction to start. Immediately after 10 minutes, the reaction solution was cooled by ice to stop the reaction. The concentration of saccharides was obtained by measuring the absorbance of the reaction solution at 490 nm. The concentration was determined using a calibration curve prepared by performing the same experiment using glucose as a standard.

### 2. Structural analysis of neutral exopolysaccharide

The neutral exopolysaccharide purified by the aforementioned anion exchange column chromatography (TOYOPEARL DEAE-650 M resin (Tosoh Corporation)) was subjected to proton-NMR and carbon-NMR, and the obtained NMR profiles are each illustrated in Fig. 3. The structural analysis results of the neutral exopolysaccharide from these NMR profiles are illustrated in Fig. 4.

From the structural analysis results, it was revealed that the neutral exopolysaccharide produced by the strain IJH-SONE68 has a structure in which N-acetylglucosamines are linked with each other via α-1,6 bond.

### 3. Saccharide composition analysis of acidic exopolysaccharide

The saccharide composition analysis of the aforementioned acidic exopolysaccharide purified by the anion exchange column chromatography was performed by measuring the composition by a high performance liquid chromatography (HPLC) method.

A 7-fold diluted sample solution was prepared by mixing 10 µL of the purified acidic exopolysaccharide (7.3 mg/mL) and 60 µL of water and placed in a test tube. 20 µL of the diluted sample solution was collected from the test tube, dried under reduced pressure, and 100 µL of 2 mol/L trifluoroacetic acid was added thereto to dissolve the dried sample. The resultant solution was substituted with nitrogen, sealed under a reduced pressure, hydrolyzed at 100°C for 6 hours, and then dried under a reduced pressure. To the obtained residue, 200 µL of water was added, dissolved, and filtrated with 0.22 µm filter, to obtain a sample solution for measurement. The sample solution for measurement was 10-fold diluted with water to obtain a sample solution for dilution measurement. 50 µL of each of these sample solutions was analyzed. HPLC system: LC-20A system (Shimadzu Corporation) and spectrofluorophotometer M-10AxL (Shimadzu Corporation) were used as analytical instruments. The analysis conditions were as follows:
Column: TSK-gel Sugar AXG 4.6 mml. D. × 15 cm (Tosoh Corporation)
Column temperature: 70°C
Mobile phase: 0.5 mol/L potassium borate buffer, pH 8.7
Mobile phase flow rate: 0.4 mL/min
Post column labeling: reaction reagent: 1 w/v% arginine • 3 w/v% boric acid
Reaction reagent flow rate: 0.5 mL/min
Reaction temperature: 150°C
Detection wavelength: Ex. 320 nm, Em. 430 nm

Chromatograms of samples prepared from the acidic exopolysaccharide and calibration curve data of each monosaccharide were obtained. The concentrations of constituent saccharides of the acidic exopolysaccharide in the samples were determined from calibration curves. The obtained results are shown in Table 2.

**[Table 2]**

| Constituent saccharides of acidic exopolysaccharide | | |
|---|---|---|
| Acidic exopolysaccharide | | Concentration in sample (mg/mL) |
| | Rhamnose | 0.0204 |
| | Ribose | n.d. |
| | Mannose | 3.43 |
| Monosaccharides | Arabinose | n.d. |
| | Galactose | 0.0384 |
| | Xylose | n.d. |
| | Glucose | 0.219 |

| | | |
|---|---|---|
| In the Table, n.d. indicates no detection. | | |

### Example 3

### Suppression action of IJH-SONE68 on mouse active cutaneous anaphylaxis

The suppression action of the lactic acid bacterium of the strain IJH-SONE68 obtained in Example 1 on mouse active cutaneous anaphylaxis was investigated according to a method disclosed below.

### 1. Test method

### (1) Preparation of sample for mouse test

*Lactobacillus paracasei* strain IJH-SONE68 was cultured in a juice medium (the final concentration of a pineapple juice: 50%, the final concentration of a sake extract: 10%) for 48 hours, and the resultant fermented broth was concentrated at two-fold to be used as a sample for mouse test. This sample was estimated to contain polysaccharides including neutral polysaccharide fractions and acidic polysaccharide fractions, which were polysaccharides produced by the strain IJH-SONE68 obtained in Example 2, at a concentration of about 40 mg/L.

### (2) Reagents used in test

Ovalbumin (OVA) (Cat. A5503-5G, Grade V) and aluminum hydroxide (Cat. 239186-25G) were purchased from SIGMA-ALDRICH, KOH, EvansBlue, phosphoric acid, and acetone were purchased from Wako Pure Chemical Industries, and distilled water and saline were purchased from Otsuka Pharmaceutical Factory.

### (3) Method

The mice used were male BALB/c AnNCrlCrlj mice (7-week old at the time of arrival, Charles River Japan, Inc.). One to five of the mice were housed in a breeding cage and bred in an animal breeding room in which environment was adjusted to temperature of 20 to 25°C, humidity of 40 to 70%, ventilation frequency of 13 times or more/hour, and lighting time of 12 hours (7:00 to 19:00). The mice were allowed to freely ingest solid feed CRF-1 (Oriental Yeast Co., Ltd.). In addition, with regard to drinking water, the mice were allowed to freely ingest filter-filtered water.

After carried in the cage, the mice were acclimated for about one week. After acclimated breeding, the mice were weighed and divided into groups so that the weight of each group was equal (Group A: a group in which water was administered to the mice; Group B: a group in which a pine juice solution fermented with the strain IJH-SONE68 was administered to the mice) (Group A: 9 mice; Group B: 9 mice). That time point was designated as Day 0, and the sample was orally administered to the mice once a day from Day 0 for 30 consecutive days. The daily dose volume was 0.75 mL/mouse. In addition, all of the mice were weighed once a week during Day 0 to Day 30. The body weight was measured before the administration of the sample.

On Day 14, an OA antigen solution (20 µg of OVA + 2 mg of aluminum hydroxide) were intraperitoneally administered to all of the mice at a dose of 0.2 mL/mouse (antigen sensitization). Thereafter, on Day 30, a 1% OVA solution was intradermally administered to the left auricle of the mice at a dose of 10 µL/mouse, followed by a tail vein administration of a 0.5% Evans blue solution at a dose of 0.25 mL/mouse. Thirty minutes later, the mice were euthanized by cervical dislocation, and after confirming death, the left and right auricles were collected.

The collected left and right auricles were each placed in a test tube, and 0.75 mL of 1N KOH was added thereto to dissolve the auricles at 37°C overnight. Then, 9.25 mL of a mixed solution in which 0.2 M phosphoric acid and acetone were mixed at a ratio of 5:13 was added thereto, followed by shaking. The resultant supernatant was transferred to a microplate and an absorbance was measured at 620 nm. The amount of pigment leakage was calculated as the difference between the amount of Evans blue in the left and right auricles. In addition, the data were shown by the average value ± standard error, and the t-test by Welch's method was used for a significant difference test among groups, and it was determined that there was a significant difference when a risk factor (p value) was less than 0.05.

The presence or absence of the suppression action of the sample on mouse active cutaneous anaphylaxis (ACA) was evaluated according to the method disclosed herein above.

### 2. Test result

Table 3 shows results of measuring the amount of Evans blue pigment leakage.

**[Table 3]**

| Pigment leakage amount in each mouse used in experiment | | |
|---|---|---|
| Mouse number | Group A (control group) | Group B (sample administration group) |
| 1 | 3.16 | 5.87 |
| 2 | 4.06 | 0.45 |
| 3 | 4.51 | 0.45 |
| 4 | 2.26 | 0.90 |
| 5 | 6.77 | 4.06 |
| 6 | 4.06 | 3.16 |
| 7 | 4.51 | 3.16 |
| 8 | 4.96 | 4.51 |
| 9 | 6.32 | 1.35 |
| Average | 4.51 | 2.66 |
| SE | 0.47 | 0.65 |

Fig. 5 also shows the obtained results. The left and right auricles were collected from each mouse, and the average value of the amount of pigment leaked from the left auricle was shown in the graph of Fig. 5 for each group. At that time, the amount of pigment leakage from the right auricle was used as a blank.

As is clear from Table 3 and Fig. 5, the pigment leakage amount was 4.51 ± 0.47 µg/mL in the control group (Group A: the water administration group) and 2.66 ± 0.65 µg/mL in the sample administration group (Group B). It was confirmed that the sample administration group showed a significant suppression action of about 41% (p < 0.05), as compared to the control group. From these results, the repeated oral administration of the pine juice solution fermented with the strain IJH-SONE68 before and after the antigen sensitization suppressed the ACA reaction in the mice sensitized with ovalbumin as an antigen. This sample is considered to have a potential to suppress the production of an antibody in an antigen-antibody reaction (IgE-dependent).

As is clear from the foregoing detailed disclosures, the present invention provides the following inventions:
[1] A composition for the prevention or improvement of type I allergy, comprising, as an active ingredient, a cell body of a lactic acid bacterium belonging to *Lactobacillus paracasei,* a cultured product or fermented product thereof, or a polysaccharide produced thereby.
[2] The composition according to the above [1], wherein the composition is for the prevention or improvement of anaphylaxis.
[3] The composition according to the above [1] or 2, wherein the lactic acid bacterium is a lactic acid bacterium derived from a fig.
[4] The composition according to any one of the above [1] to [3], wherein the lactic acid bacterium is *Lactobacillus paracasei* strain IJH-SONE68 (Accession No. NITE BP-02242) or a lactic acid bacterium equivalent thereto.
[5] The composition according to any one of the above [1] to [4], wherein the cultured product or fermented product of the lactic acid bacterium is a cultured product or fermented product that is obtained by cultivating or fermenting the lactic acid bacteria in the presence of a fruit juice of pineapple genus plant.
[6] The composition according to any one of the above [1] to [4], wherein the polysaccharide is a neutral polysaccharide having a structure in which N-acetylglucosamines are linked with each other via α-1,6 bond.
[7] The composition according to any one of the above [1] to [4], wherein the polysaccharide is an acidic polysaccharide composed mainly of glucoses and mannoses.
[8] The composition according to any one of the above [1] to [7], wherein the composition is a food or drink composition.
[9] The composition according to the above [8], wherein the food or drink is a beverage, a functional food, a fermented food, or a supplement.
[10] The composition according to any one of the above [1] to [7], wherein the composition is a pharmaceutical composition.
[11] The composition according to any one of the above [1] to [7], wherein the composition is a feed composition.
[12] The composition according to any one of the above [1] to [7], wherein the composition is a cosmetic composition.
[13] Use of a cell body of a lactic acid bacterium belonging to *Lactobacillus paracasei,* a cultured product or fermented product thereof, or a polysaccharide produced thereby, as an active ingredient of a composition for the prevention or improvement of type I allergy.
[14] The use according to the above [13], wherein the composition is for the prevention or improvement of anaphylaxis.
[15] The use according to the above [13] or [14], wherein the lactic acid bacterium is a lactic acid bacterium derived from a fig.
[16] The use according to any one of the above [13] to [15], wherein the lactic acid bacterium is *Lactobacillus paracasei* strain IJH-SONE68 (Accession No. NITE BP-02242) or a lactic acid bacterium equivalent thereto.
[17] The use according to any one of the above [13] to [16], wherein the cultured product or fermented product of the lactic acid bacterium is a cultured product or fermented product that is obtained by cultivating or fermenting the lactic acid bacteria in the presence of a fruit juice of pineapple genus plant.
[18] The use according to any one of the above [13] to [16], wherein the polysaccharide is a neutral polysaccharide having a structure in which N-acetylglucosamines are linked with each other via α-1,6 bond.
[19] The use according to any one of the above [13] to [16], wherein the polysaccharide is an acidic polysaccharide composed mainly of glucoses and mannoses.
[20] The use according to any one of the above [13] to [19], wherein the composition is a food or drink composition.
[21] The use according to the above [20], wherein the food or drink is a beverage, a functional food, a fermented food, or a supplement.
[22] The use according to any one of the above [13] to [19], wherein the composition is a pharmaceutical composition.
[23] The use according to any one of the above [13] to [19], wherein the composition is a feed composition.
[24] The use according to any one of the above [13] to [19], wherein the composition is a cosmetic composition.
[25] A method for applying a composition comprising, as an active ingredient, a cell body of a lactic acid bacterium belonging to *Lactobacillus paracasei,* a cultured product or fermented product thereof, or a polysaccharide produced thereby, to a subject in need thereof, wherein the method prevents or improves type I allergy in the subject.
[26] The method according to the above [25], wherein the method prevents or improves anaphylaxis.
[27] The method according to any one of the above [25] to [26], wherein the lactic acid bacterium is a lactic acid bacterium derived from a fig.
[28] The method according to any one of the above [25] to [27], wherein the lactic acid bacterium is *Lactobacillus paracasei* strain IJH-SONE68 (Accession No. NITE BP-02242) or a lactic acid bacterium equivalent thereto.
[29] The method according to any one of the above [25] to [28], wherein the cultured product or fermented product of the lactic acid bacterium is a cultured product or fermented product that is obtained by cultivating or fermenting the lactic acid bacteria in the presence of a fruit juice of pineapple genus plant.
[30] The method according to any one of the above [25] to [28], wherein the polysaccharide is a neutral polysaccharide having a structure in which N-acetylglucosamines are linked with each other via α-1,6 bond.
[31] The method according to any one of the above [25] to [28], wherein the polysaccharide is an acidic polysaccharide composed mainly of glucoses and mannoses.
[32] The method according to any one of the above [25] to [31], wherein the composition is a food or drink composition.
[33] The method according to the above [32], wherein the food or drink is a beverage, a functional food, a fermented food, or a supplement.
[34] The method according to any one of the above [25] to [31], wherein the composition is a pharmaceutical composition.
[32] The method according to any one of the above [25] to [31], wherein the composition is a feed composition.
[33] The method according to any one of the above [25] to [31], wherein the composition is a cosmetic composition.

### Industrial Applicability

As disclosed in detail herein above, the composition of the present invention is effective in preventing or improving type I allergy and can be used to prevent or improve particularly anaphylaxis. The composition of the present invention can be used as a food or drink, a medicine, a feed, and a cosmetic product, for the prevention or improvement of type I allergy including anaphylaxis. The composition of the present invention comprises, as an active ingredient, a cell body of a lactic acid bacterium belonging to *Lactobacillus paracasei,* a cultured product or fermented product thereof, or a polysaccharide produced thereby. Therefore, the composition of the present invention has high safety, can be applied for a long period of time, can be supplied in a large amount at low cost, and has extremely high utility and practicality.

## Claims

1. A composition for the prevention or improvement of type I allergy, comprising, as an active ingredient, a cell body of a lactic acid bacterium belonging to *Lactobacillus paracasei,* a cultured product or fermented product thereof, or a polysaccharide produced thereby.

2. The composition according to claim 1, wherein the composition is for the prevention or improvement of anaphylaxis.

3. The composition according to claim 1 or 2, wherein the lactic acid bacterium is a lactic acid bacterium derived from a fig.

4. The composition according to any one of claims 1 to 3, wherein the lactic acid bacterium is *Lactobacillus paracasei* strain IJH-SONE68 (Accession No. NITE BP-02242) or a lactic acid bacterium equivalent thereto.

5. The composition according to any one of claims 1 to 4, wherein the cultured product or fermented product of the lactic acid bacterium is a cultured product or fermented product that is obtained by cultivating or fermenting the lactic acid bacteria in the presence of a fruit juice of pineapple genus plant.

6. The composition according to claims 1 to 4, wherein the polysaccharide is a neutral polysaccharide having a structure in which N-acetylglucosamines are linked with each other via α-1,6 bond.

7. The composition according to any one of claims 1 to 4, wherein the polysaccharide is an acidic polysaccharide composed mainly of glucoses and mannoses.

8. The composition according to any one of claims 1 to 7, wherein the composition is a food or drink composition.

9. The composition according to claim 8, wherein the food or drink is a beverage, a functional food, a fermented food, or a supplement.

10. The composition according to any one of claims 1 to 7, wherein the composition is a pharmaceutical composition.

11. The composition according to any one of claims 1 to 7, wherein the composition is a feed composition.

12. The composition according to any one of claims 1 to 7, wherein the composition is a cosmetic composition.
